# EUROPEAN PATENT APPLICATION

(11) **EP 1 489 134 A1**
(43) Date of publication of application: **22.12.2004**
(21) Application number: 04012949.6
(22) Date of filing: 01.06.2004
(51) Int. Cl.: C08K 3/22, A61F 13/00, D04H 1/00

(54) **X-ray detectable fiber and medical absorbent gauze including the same**

(30) Priority: 16.06.2003 JP 2003170755
(71) Applicant: Nisshinbo Industries, Inc., Chuo-ku, Tokyo 103-8650 (JP)
(72) Inventor: Sugiyama, Koju, 4-chome, Fukushima-ku Osaka 553-0007 (JP); Yamaguchi, Kaoru, 4-chome, Fukushima-ku, Osaka 553-0007 (JP); Osada, Tomoki, 4-chome, Fukushima-ku, Osaka 553-0007 (JP); Fukuda, Jirou, c/o Nisshinbo Industries, Inc, Okazaki-shi Aichi 444-0803 (JP)
(74) Representative: Behnisch, Werner, Dr.

(57) **Abstract**

An X-ray-detectable fiber contains a radiopaque inorganic substance and a natural vegetable fiber. The X-ray detectable fiber is characterized in that the radiopaque inorganic substance is contained in a substantial portion of the natural vegetable fiber, and that the content of the radiopaque inorganic substance in the X-ray-detectable fiber is 21 to 50 percent by weight.

## Description

### BACKGROUND OF THE INVENTION

### 1. Field of the Invention

The present invention relates to X-ray-detectable fibers and medical absorbent gauzes including the X-ray-detectable fibers.

### 2. Description of the Related Art

Surgical procedures require the use of a large number of gauze pieces and sponges to absorb blood during the procedure. Blood-saturated gauzes and sponges can be easily overlooked and may remain in the body after the completion of the surgical procedures. In order to locate gauzes and sponges that have been left in the body with X-ray, gauzes and sponges with radiopaque components, e.g., X-ray detectable threads, are commonly used. An example is a gauze containing an X-ray detectable thread composed of vinyl chloride and barium sulfate kneaded into the vinyl chloride (Japanese Unexamined Utility Model Registration Application Publication No. 4-104824). However, this gauze suffers from detachment and folding of the threads, and such threads disadvantageously remain inside the body. While gauzes and sponges are disposable and discarded after being used once, it is not preferable from the environmental standpoint for gauzes to contain synthetic polymers, such as polyesters, which generate large amounts of heat and have low biodegradability.

Gauzes and sponges are also used to remove blood and body fluids from the body without significantly exposing affected areas. Examples of such medical treatments include abdominal operations such as drainage and tamponade. Drainage refers to insertion of gauze drains composed of antibiotic-impregnated gauzes or sponges into affected areas using Sondes so as to discharge body fluids, such as blood, from exposed portions of the gauze drains outside the body by capillary action. Tamponade refers to insertion of gauze drains to affected areas so as to allow the gauze drains to absorb blood or other body fluids. Both drainage and tamponade require intricate operational procedures and are challenging even for skilled and experienced doctors.

Drainage or tamponade operations would be greatly simplified if gauze drains composed of X-ray detectable gauzes and sponges, which can be identified by X-ray imaging, were available. However, conventional X-ray detectable gauzes or sponges contain thick, hard X-ray detectable threads and cannot be easily inserted or handled. Moreover, since only the X-ray detectable threads appear in the X-ray image, the exact location of the gauze drain is rarely identified.

In order to overcome these problems, Japanese Unexamined Patent Application Publication No. 7-102459 describes a nonwoven fabric prepared by forming a web from a hydrophilic fiber, such as rayon, containing radiopaque barium sulfate using a conventional technique and then entangling the fiber by high-pressure water jetting. However, in order to make this nonwoven fabric, the hydrophilic fiber must be dissolved or melted to allow sufficient incorporation of barium sulfate. Thus, according to this method, it is essentially impossible to introduce radiopaque substances, such as barium sulfate, into natural vegetable fibers such as cotton, which is the most suitable material for gauze applications.

PCT Japanese Translation Patent Publication No. 8-502328 describes a method for introducing an inorganic filler to dry cellulose fibers and a composition containing barium sulfate and barium silicate for use in this method. However, the maximum filler content is only 20 percent by weight under the most preferable conditions. This method includes the steps of adding an alkaline first solution and adding a neutral or acidic second solution.

Japanese Patent No. 3317660 teaches another technique for introducing an inorganic substance into the substantial portion of a natural vegetable fiber by utilizing swelling of fibers in an alkaline solution.

Cellulose, a major constituent of vegetable fibers, is generally classified into α-, β-, and γ-cellulose according to the molecular weight. Since high-molecular-weight α-cellulose is alkali-insoluble and almost all inorganic substances are alkali-stable, swelling of vegetable fibers is generally carried out by alkaline treatments.

On the other hand, in the preparation of cellulose derivatives, an acid is used to induce swelling and chemical reaction. For example, acids are widely used in producing acetylcellulose and nitrocellulose. However, acid treatments significantly decrease the molecular weight of the cellulose, resulting in a lower production yield when compared to alkaline treatments. Furthermore, decomposition of many inorganic substances occurs during acid treatments. Thus, acid treatments are not as widely applied to induce the swelling of cellulose as the alkaline treatments.

### SUMMARY OF THE INVENTION

An object of the present invention is to provide a high-safety X-ray-detectable fiber, which can be adequately detected by X-rays, has superior water absorbing and retention properties and sufficient strength, and can be easily incinerated or biologically decomposed once discarded. The X-ray-detectable fiber is particularly suitable for medical absorbent gauze applications. A medical absorbent gauze including the X-ray-detectable fiber is also provided.

To achieve this object, a first aspect of the present invention provides an X-ray-detectable fiber containing a radiopaque inorganic substance and a natural vegetable fiber, characterized in that the radiopaque inorganic substance is contained in a substantial portion of the natural vegetable fiber and that the content of the radiopaque inorganic substance in the X-ray-detectable fiber is 21 to 50 percent by weight.

Preferably, the radiopaque inorganic substance contains at least one selected from the group consisting of barium sulfate, barium aluminosilicate, and barium silicate.

The X-ray detectable fiber of the present invention contains a sufficient amount of the radiopaque inorganic substance to achieve high X-ray detectability while achieving sufficient fiber strength. Since the fiber component of the X-ray detectable fiber is a natural vegetable fiber, the X-ray detectable fiber exhibits superior water absorption and retention properties, is highly safe, and can be easily incinerated or biologically decomposed when discarded. Thus, the X-ray detectable fiber of the present invention is suitable for medical absorbent gauze applications.

A second aspect of the present invention provides a method for producing the X-ray-detectable fiber. The method includes the step of swelling the natural vegetable fiber in a first solution containing an acid or a salt thereof; and adding a second solution containing a metal ion capable of forming the radiopaque inorganic substance with the acid.

Preferably, the first solution contains an acid.

A third aspect of the present invention provides a medical absorbent gauze containing the X-ray-detectable fiber.

Preferably, the medical absorbent gauze contains 40 to 100 percent by weight of the X-ray-detectable fiber and 0 to 60 percent by weight of a natural vegetable fiber.

The medical absorbent gauze of the present invention has good X-ray detectability. Thus, the exact location of the gauze can be identified in the case where the gauze remains inside the body after surgery or during drainage operations with X-ray imaging.

### DESCRIPTION OF THE PREFERRED EMBODIMENTS

For the purposes of the present invention, the term "radiopaque inorganic substance" refers to an inorganic substance capable of forming a contrast image in a radiograph. Examples of such inorganic substances include barium sulfate, barium aluminosilicate, and barium silicate. Barium sulfate is particularly preferred since it is X-ray detectable in smallest doses. In the present invention, the radiopaque inorganic substances may be used alone or in combination.

In the present invention, the term "natural vegetable fiber" refers to a fiber mainly composed of cellulose derived from vegetables in the course of spinning or paper-manufacturing, for example. Examples of such fibers include natural cellulose fibers such as pulp, kenaf, cotton, hemp, straw, and rice straw; and calcium alginate fibers extracted from seaweed. The natural vegetable fiber may partly contain rayon fibers. Cotton is preferred as the material for medical absorbent gauzes since it has superior water absorption and retention properties and is highly safe. The form of the fiber is not particularly limited. The fiber may be provided in the form of filaments, nonwoven fabric, knit, or woven textile. The natural vegetable fiber may be used alone or in combination with other type or types of natural vegetable fiber.

In the present invention, the radiopaque inorganic substance is contained in the substantial portion of a natural vegetable fiber.

The statement "in the substantial portion of a natural vegetable fiber" means "at the interior of the structure composed of natural cellulose" when the natural vegetable fiber is a natural cellulose fiber such as pulp. In particular, this means that a substance concerned is either in a site (100 to 5,000 Å) produced by swelling between microfibrils (about 0.1 µm in diameter) of cell walls or in a site produced by swelling of an amorphous region in a micelle of a microfibril. The surface of the cell walls composed of cellulose, pores originally present in the cell walls, and lumens are not included in the substantial portion of the natural vegetable fiber.

Moreover, the statement "a radiopaque inorganic substance is contained in the substantial portion of a natural vegetable fiber" means that the radiopaque inorganic substance is at least partly contained in the substantial portion of the cellulose.

In the present invention, the substantial portion of the natural vegetable fiber contains 21 to 50 percent by weight, and preferably 25 to 45 percent by weight of a radiopaque inorganic substance. Fibers containing less than 21 percent by weight of the radiopaque inorganic substance in the substantial portion have liquid absorption properties comparable to conventional gauzes but do not have sufficient X-ray detectability and would cause the same medical problems as in the known art. Fibers containing more than 50 percent by weight of the radiopaque inorganic substance in the substantial portion do not have sufficient strength. As a result, fiber fragments or gauze fragments may become detached and remain inside the body. Fibers containing the radiopaque substance in an amount within the range described above have sufficient X-ray detectability, exhibit sufficient strength, and are easy to fabricate.

The X-ray-detectable fiber of the present invention may be fabricated by either of the following two methods:
1. A method for producing a radiopaque inorganic substance in the substantial portion of a natural vegetable fiber, including the steps of swelling a natural vegetable fiber in a solution containing an acid or a salt thereof; and adding a solution containing a metal ion capable of producing the radiopaque inorganic substance by the reaction with the acid. In this method, the inorganic substance is obtained as a deposit; and
2. A cation exchange method including a step of exchanging a cation in aluminosilicate or silicate with a metal ion, such as a barium ion, capable of forming a radiopaque inorganic substance with the aluminosilicate or the silicate, in which an aluminosilicate or silicate-natural vegetable fiber compound material containing cation-exchangeable, acid-resistant aluminosilicate or silicate in the substantial portion of a natural vegetable fiber prepared by, for example, the method described in Japanese Patent No. 3317660 is used as a starting material.

These methods will now be described in detail.

### Method 1

In method 1, the acid or the salt thereof contained in the solution (this solution is hereinafter simply referred to as "first solution") is not particularly limited as long as it swells the natural vegetable fiber and forms a radiopaque inorganic substance by the reaction with a metal ion contained in another solution described below. For obtaining fibers containing a large amount of, i.e., 21 percent by weight or more of, radiopaque substance, sulfuric acid and its salts are preferred since they are widely used as the swelling agent for natural vegetable fibers. The swellability of natural vegetable fibers is higher with acids than with salts of the acids. In the present invention, an acid, in particular, sulfuric acid, is preferably used.

Examples of the salt include soluble metal salts such as sodium salts. Sodium salts, in particular sodium sulfate, are preferred. The solvent may be any but must not inhibit the synthesis of the radiopaque inorganic substance. Water may be used as the solvent, for example.

The concentration of the acid or its salt in the first solution is not particularly limited as long as the molecular weight of the natural vegetable fiber is not reduced. The preferable ranges that can achieve sufficient swelling without decreasing the molecular weight are 0.5 to 20 (w/v)% and more preferably 1.0 to 10 (w/v)%.

In method 1, the solution containing a metal ion capable of forming a radiopaque inorganic substance (hereinafter this solution is simply referred to as "second solution") may be any as long as the metal ion contained can react with the acid in the first solution to produce the radiopaque inorganic substance. The second solution may be selected according to the first solution used. For example, when the first solution contains sulfuric acid or its salt, a second solution containing barium ion is preferably used to produce barium sulfate.

The metal ion is generally derived by dissolving a metal salt in the solution. For example, in order to obtain barium ion, a soluble metal salt, such as barium chloride, barium acetate, or barium nitrite, may be used. Barium chloride having high solubility is particularly preferred. The solvent in the second solution is not particularly limited but must not inhibit the formation of the radiopaque inorganic substance. In general, water may be used as the solvent.

The metal ion concentration in the second solution is not particularly limited and is preferably selected according to the type of metal ion used. A solution containing a barium ion as the metal ion is typically prepared by dissolving a barium salt. The barium salt concentration is preferably adjusted to 1.0 to 50 (w/v)%, and more preferably 2.0 to 40 (w/v)% to prevent the formation of excess barium sulfate outside the fibers.

The swelling of the natural vegetable fiber using the first solution is usually conducted by immersion of the natural vegetable fiber into the first solution. The immersion time is not particularly limited but needs to be sufficiently long to allow the first solution to reach the interior of the fiber. The immersion time is preferably 5 minutes to 3 days, and more preferably 10 minutes to 1 day so that the molecular weight of the natural vegetable fibers, e.g., cellulose, does not decrease during the acidic treatment.

The immersion temperature is preferably 60°C or less since the molecular weight of the natural vegetable fibers (e.g., cellulose) tends to decrease at higher temperatures. Preferably, the immersion temperature is within the range of 10 to 50°C to sufficiently swell fibers without decomposition.

Prior to adding the second solution, the excess first solution may be removed from the reaction system by centrifugation or mechanical squeezing so as to avoid the undesirable formation of the radiopaque inorganic substance (e.g., barium sulfate) outside the fibers. Centrifugation is preferably performed at 2,000 to 5,000 rpm for 1 to 30 minutes. Mechanical squeezing is preferably performed at 10 to 50 kgf/cm². Centrifugation is more advantageous for introducing the radiopaque inorganic substance (e.g., barium sulfate) to filamentous fibers. Mechanical squeezing is more advantageous for fibers in the form of a nonwoven fabric, knit, or woven textile.

The second solution may be added at any suitable time but is preferably added immediately after the removal of the first solution since the molecular weight of the natural vegetable fiber (e.g., cellulose) is high at this stage.

The reaction temperature after the addition of the second solution is preferably 60°C or less to prevent decomposition of the natural vegetable fiber (e.g., cellulose). The reaction temperature is preferably 10 to 50°C to prevent a decrease in the molecular weight of the natural vegetable fiber (e.g., cellulose).

The reaction time after the addition of the second solution is not particularly limited but is preferably 5 minutes to 3 days to allow the formation of a target radiopaque inorganic substance (e.g., barium sulfate) by the reaction between an acid, e.g., sulfuric acid, and a metal ion, e.g., a barium ion. The reaction time is preferably 10 minutes to 1 day to prevent a decrease in the molecular weight of the natural vegetable fibers, e.g., cellulose.

After of the reaction, the fiber is washed by, for example, a known method. In particular, the fiber is placed in a centrifugal machine and washed by continuously supplying water into the rotating centrifugal machine; alternatively, the fiber may be dispersed in water contained in a tank and then dehydrated.

In method 1, the step of swelling the natural vegetable fiber using the first solution and the subsequent step of adding the second solution to form the radiopaque inorganic substance may be repeated a number of times. In particular, these steps are preferably repeated until a sufficient amount of radiopaque inorganic substance is retained in the fiber.

### Method 2

An aluminosilicate or silicate-natural vegetable fiber compound material containing cation-exchangeable, acid-resistant aluminosilicate or silicate in the substantial portion of the natural vegetable fiber is known in the art. This compound material can be prepared by the method disclosed in Japanese Patent No. 3317660.

In particular, for making a zeolite-pulp compound material, pulp is swelled in an aqueous solution of 10 to 50,000 mmol/l of a basic substance, e.g., sodium hydroxide, and an aqueous solution of 1.0 to 10,000 mmol/l of an aluminum compound, e.g., sodium aluminate is added. An aqueous solution of 1.0 to 1,000 mmol/l of a silicon compound, e.g., sodium metasilicate, is subsequently added, and the mixture is reacted at 20 to 100°C for 1 hour to 20 days to obtain the zeolite-pulp compound material.

For introducing a metal ion, e.g., a barium ion, into aluminosilicate or silicate in the substantial portion of the natural vegetable fiber through cation exchange, a known cation exchange method, such as the method disclosed in Japanese Patent No. 3317660, may be employed. When the metal salt, e.g., barium salt, used in the cation exchange is water-soluble, the reaction temperature and the reaction time are not particularly limited. For example, in conducting cation exchange in an aqueous solution, the metal ion concentration in the aqueous solution is normally 0.001 to 10 mmol/1 and preferably 0.01 to 5 mol/1. The salt from which the metal ion is derived is not particularly limited as long as it produces the desired metal ion. For example, when a barium ion is desired, barium chloride, barium acetate, barium nitrite, or the like may be used. The temperature of the aqueous solution and the immersion time may be adequately determined based on the properties and the content of the aluminosilicate or the silicate, the properties and the concentration of the metal salt, and the cation exchange efficiency. The temperature is normally 0 to 100°C and preferably 20 to 50°C. The immersion time is normally 2 to 10 hours, and preferably 10 to 48 hours.

The X-ray-detectable fiber of the present invention is composed of a soft natural vegetable fiber containing the radiopaque substance. Thus, the fiber is easy to process and can be worked into various forms, e.g., filaments, nonwoven fabrics, knit, woven textiles, particles, cubes, and sheets, depending on the needs. Since the X-ray-detectable fiber of the present invention can be manufactured without changing the form of the starting material, i.e., the natural vegetable fiber, it is preferable to process the natural vegetable fiber to have a desired form prior to introducing the radiopaque inorganic substance into the fiber.

The X-ray-detectable fiber of the present invention containing the above-described amount of the radiopaque substance has both sufficient fiber strength and X-ray detectability. Since the fiber component of the X-ray-detectable fiber of the present invention is a natural vegetable fiber, the X-ray-detectable fiber achieves superior water absorbing and retention properties and high safety, and can be easily incinerated or biologically decomposed once discarded. Thus, the X-ray-detectable fiber of the present invention is particularly suitable for medical absorbent gauze applications.

The X-ray-detectable fiber of the present invention may contain a pharmaceutical liquid, such as an antibacterial agent, disinfectant alcohol, an iodine solution, or oxydol, if desired.

A medical absorbent gauze of the present invention contains the X-ray-detectable fiber described above. The content of the X-ray-detectable fiber in the medical absorbent gauze depends on the radiopaque substance content in the X-ray-detectable fiber. When the radiopaque substance content in the X-ray-detectable fiber is 21 percent by weight, the content of the X-ray-detectable fiber in the medical absorbent gauze is preferably about 100 percent by weight. When the radiopaque substance content in the X-ray-detectable fiber is 50 percent by weight, the content of the X-ray-detectable fiber in the medical absorbent gauze is preferably about 40 percent by weight. The medical absorbent gauze of the present invention preferably contains 40 to 100 percent by weight of the X-ray-detectable fiber and 0 to 60 percent by weight of a natural vegetable fiber to achieve sufficient water absorption and retention properties, high safety, and sufficient strength. More preferably, the medical absorbent gauze contains 50 to 90 percent by weight of the X-ray-detectable fiber and 10 to 50 percent by weight of a natural vegetable fiber. The natural vegetable fiber contained in the gauze is preferably the same as the starting material of the X-ray-detectable fiber to obtain sufficient strength for entanglement and water absorption and retention properties required in actual applications.

The medical absorbent gauze of the present invention may be prepared by a known method. For example, when the X-ray-detectable fiber is provided in the form of a nonwoven fabric, the nonwoven fabric is first disintegrated and mixed with a natural vegetable fiber, and the mixed fibers are entangled by a high-pressure water jet. The pressure of the water jet is typically 50 to 100 kg/cm².

The medical absorbent gauze of the present invention containing the above-described X-ray-detectable fiber shows sufficient X-ray detectability. Thus, the accurate position of the medical absorbent gauze can be detected in the case where the gauze is left inside the body after surgery or when drainage is carried out with X-ray imaging.

Moreover, when a conventional medical absorbent gauze having interwoven X-ray detectable threads in vertical and horizontal directions is cut into small pieces, some pieces may not contain X-ray detectable threads. The medical absorbent gauze of the present invention does not suffer from such a problem.

In medical practice, products that can be used in the same manner as the conventional products are preferred. The X-ray detectable medical absorbent gauze of the present invention maintains the properties of the natural vegetable fiber and thus can be used in the same manner as conventional medical absorbent gauzes.

The medical absorbent gauze of the present invention may contain a pharmaceutical liquid, such as an antibacterial agent, disinfectant alcohol, an iodine solution, or oxydol, if desired.

### EXAMPLES

The present invention will now be described by way of nonlimiting examples.

### Example 1

Twenty-two grams of cotton (absorbent cotton listed in Japanese Pharmacopoeia, manufactured by Marusan Industrial Co., Ltd.) was weighed, and 50 ml of a sulfuric acid solution (sulfuric acid:water = 1:8) was added. The cotton was left to stand for 24 hours at room temperature to swell the fibers. The resulting fibers were dewatered in a small centrifugal dehydrator (SYK-5000 manufactured by Sanyo Rikagaku Kikai Sesisakusho Kabushikikaisha) rotating at 2,000 rpm for 1 minute. Subsequently, 100 mol of a 40 (w/v)% barium chloride aqueous solution was added, and the fibers were left to stand still at room temperature (15 to 25°C) for 24 hours to carry out the reaction. The resulting fiber was washed in the centrifugal dehydrator with 2,000 mL of water to remove barium sulfate generated outside the fibers.

The barium sulfate content in the resulting fibers was determined as: (the ash content of 1 g of the resulting fiber after calcination at 400°C)/(initial weight). The barium sulfate content was 25.1 percent by weight.

### Example 2

Twelve grams of a cotton nonwoven fabric (product name: Oikos AP2050, 50 g/m² in weight, manufactured by Nisshinbo Industries, Inc.) was weighed and immersed in 70 ml of a mixed aqueous solution containing 21 g of 48 (w/v)% caustic soda (manufactured by Toagosei Co., Ltd.) and 15 g of liquid sodium aluminate (NA170, manufactured by Sumitomo Chemical Co., Ltd.). After adding 11 g of sodium silicate (No. 1-L2, manufactured by Toso Sangyo Co., Ltd.), the mixture was heated to 50°C, and 80 mL of a 10% sodium silicate aqueous solution was added. The resulting nonwoven fabric impregnated with the solution was enclosed in a container to avoid drying and then heated for 4 hours in a dryer (DP32, manufactured by Sibata Scientific Technology Ltd.) set to 90° C to obtain 17.9 g of a cotton nonwoven fabric containing 44.1 percent by weight of zeolite X in the substantial portion of the cotton fibers. The nonwoven fabric was thoroughly washed and dried. The dried nonwoven fabric was then immersed in 5,000 ml of a barium nitrite aqueous solution (barium concentration: 0.06 (w/v)%) at room temperature for 24 hours to conduct cation exchange.

Elemental analysis was conducted with an X-ray fluorescence element analyzer (MESA-500, manufactured by Horiba Ltd.). The cotton nonwoven fabric contained 129 mg/g of barium, and 98.5% of the cation exchange capacity of the zeolite X in the cotton nonwoven fabric was replaced with barium. The zeolite X in the cotton nonwoven fabric was barium aluminosilicate. The weight of the obtained barium-aluminosilicate-containing cotton nonwoven fabric was 19.5 g (approximately 100 g/m² in weight), and the barium aluminosilicate content in the nonwoven fabric was 48.9 percent by weight.

### Example 3

Twenty-two grams of cotton (absorbent cotton listed in Japanese Pharmacopoeia, manufactured by Marusan Industrial Co., Ltd.) was weighed, and 50 ml of a 40 (w/v)% sodium sulfate aqueous solution was added. The fibers were left to stand for 24 hours at room temperature. The resulting fibers were placed in a small centrifugal dehydrator (SYK-5000 manufactured by Sanyo Rikagaku Kikai Sesisakusho Kabushikikaisha) and dewatered at 2,000 rpm for 1 minute. Subsequently, 100 mol of a 40 (w/v)% barium chloride aqueous solution was added, and the fibers were left to stand still at room temperature for 24 hours to conduct the reaction. The fibers were washed with 2,000 mL of water in the centrifugal dehydrator to remove barium sulfate generated outside the fibers.

To the resulting fibers, 50 ml of a 40 (w/v)% barium chloride aqueous solution was again added and the fibers were left to stand at room temperature for 24 hours. The fibers were dewatered in a small centrifugal dehydrator (SYK-5000 manufactured by Sanyo Rikagaku Kikai Sesisakusho Kabushikikaisha) rotating at 2,000 rpm for 1 minute. Subsequently, 100 mol of a 40 (w/v)% barium chloride aqueous solution was added, and the fibers were left to stand still at room temperature (15 to 25°C) for 24 hours to carry out the reaction. The resulting fibers were washed with 2,000 mL of water in the centrifugal dehydrator to remove barium sulfate generated outside the fiber.

The barium sulfate content in the resulting fiber was determined as: (the ash content of 1 g of the resulting fiber after calcination at 400°C)/(initial weight). The barium sulfate content was 22.1 percent by weight.

### Example 4

The nonwoven fabric obtained in Example 2 was disintegrated and mixed with a regular cotton at a 50:50 ratio. The mixed fibers were processed with an 80 kg/cm² high-pressure water jet a number of times and formed into a nonwoven fabric having a weight of 100 g/m².

### Comparative Example 1

A viscose solution of cellulose (cellulose concentration: 9 (w/v)%) was mixed with barium sulfate. The barium sulfate content was 80 parts by weight relative to 100 parts by weight of cellulose. The mixture was thoroughly mixed and degassed. The mixture was spun in a coagulation bath by a common wet spinning method to obtain rayon containing 44 percent by weight of barium sulfate. The rayon fibers were disintegrated by a common method to form a web, and 80 kg/cm² high-pressure water jetting was performed a number of times to form a nonwoven fabric from the web. The nonwoven fabric weighed 100 g/m².

### Comparative Example 2

Cotton fibers were formed into a nonwoven fabric (Oikos AP2100) having a weight of 120 g/m² by performing 80 kg/cm² high-pressure water jetting a number of times.

### Experimental Example 1

The water absorption and retention properties of the nonwoven fabrics of Examples 2 and 4 and Comparative Examples 1 and 2 were examined.

The water absorption property was determined by the Byreck method (JIS L1096 6.26.1 Method B). In particular, a 2.5 x 20 [cm] sample was suspended so that the bottom end was dipped in physiological saline by 1 cm. The increase in the height of water resulting from capillary action was measured after one minute, 5 minutes, and 10 minutes.

The water retention property was also determined. In particular, a 10 × 10 [cm] sample was immersed in physiological saline for one minute, discharged, suspended for 30 seconds, and weighed. The difference between the weight before the immersion and the weight after the suspension was calculated. The results of the experiments are shown in Table 1.

**Table 1**

| | Example 2 | | Example 4 | | Comparative Example 1 | | Comparative Example 2 | |
|---|---|---|---|---|---|---|---|---|
| | Warp | Weft | Warp | Weft | Warp | Weft | Warp | Weft |
| 1 min | 70 | 58 | 71 | 59 | 40 | 24 | 72 | 60 |
| 5 min | 91 | 80 | 105 | 85 | 77 | 45 | 106 | 88 |
| 10 min | 120 | 109 | 121 | 111 | 117 | 82 | 122 | 112 |
| Water retention rate (%) | 632.4 | | 745.5 | | 490.4 | | 830.5 | |

### Experimental Example 2

The X-ray detectability of the nonwoven fabrics of Examples 1, 2, and 4 and Comparative Examples 1 and 2 were evaluated. Each nonwoven fabric was placed on a 15-cm acrylic phantom. X-ray irradiation was performed at 80 kV and 70 mA for 0.5 second at a distance between the X-ray source and the phantom of 120 cm. The results are shown in Table 2.

**Table 2**

| Example 1 | Example 2 | Example 4 | Comparative Example 1 | Comparative Example 2 |
|---|---|---|---|---|
| B | A | B | A | C |
| X-ray detectability: A: Excellent B: Fair C: Poor | | | | |

## Claims

1. An X-ray-detectable fiber comprising:
a radiopaque inorganic substance, and
a natural vegetable fiber,
**characterized in that** the radiopaque inorganic substance is contained in a substantial portion of the natural vegetable fiber, and that the content of the radiopaque inorganic substance in the X-ray-detectable fiber is 21 to 50 percent by weight.

2. The X-ray-detectable fiber according to claim 1, wherein the radiopaque inorganic substance comprises at least one selected from the group consisting of barium sulfate, barium aluminosilicate, and barium silicate.

3. A method for producing the X-ray-detectable fiber according to claim 1, the method comprising:
swelling the natural vegetable fiber in a first solution containing an acid or a salt thereof; and
adding a second solution containing a metal ion capable of forming the radiopaque inorganic substance with the acid.

4. The method according to claim 3, wherein the first solution contains an acid.

5. A medical absorbent gauze comprising the X-ray-detectable fiber of claim 1.

6. The medical absorbent gauze according to claim 5, comprising 40 to 100 percent by weight of the X-ray-detectable fiber of claim 1 and 0 to 60 percent by weight of a natural vegetable fiber.
